# EUROPEAN PATENT APPLICATION

(11) **EP 4 578 962 A1**
(43) Date of publication of application: **02.07.2025**
(21) Application number: 23857775.3
(22) Date of filing: 25.08.2023
(51) Int. Cl.: C12Q 1/6886, C12Q 1/6827

(54) **COMPOSITION FOR AMPLIFYING FLT3 GENE, AND USES THEREOF**

(30) Priority: 25.08.2022 KR 20220106988
(71) Applicant: Ngenebio, Seoul 08390 (KR)
(72) Inventor: KWON, Bit Na, Seoul 02625 (KR); KIM, Ji Yeon, Bucheon-si Gyeonggi-do 14581 (KR); KIM, Myung-Shin, Seoul 06668 (KR); KIM, Yong-Goo, Seoul 07982 (KR); LEE, Jong-Mi, Seoul 06544 (KR)
(74) Representative: Mewburn Ellis LLP
(86) International application number: PCT/KR2023/012622
(87) International publication number: WO 2024/043743

(57) **Abstract**

The present invention relates to a composition for amplifying a FLT3 gene, and uses thereof, and, more particularly, to a composition comprising a primer set capable of simultaneously amplifying an ITD detection region and a TKD mutation region of the FLT3 gene, and uses thereof. The composition for gene amplification, according to the present invention, enables the simultaneous performance of: diagnosis of acute myeloid leukemia (AML) in patients having FLT3-ITD mutations; determination of targeted anticancer treatment prescription for AML patients having FLT3-ITD mutations; detection of minimal residual disease (MRD) in AML patients; prognosis prediction in AML patients; and identification of drug resistance to AML tyrosine kinase inhibitors, and thus, shortens the time to derive analysis results from samples and enables efficient testing. The present invention enables the selection of correct and rapid diagnosis and treatment methods in the treatment of patients with acute myeloid leukemia, and thus is useful for early treatment and recurrence prevention.

## Description

### [Technical Field]

The present invention relates to a composition for amplifying FLT3 genes and the use thereof, more specifically, to a composition containing primer sets for simultaneously amplifying an ITD detection region and a TKD mutation region of FLT3 genes and the use thereof.

### [Background Art]

90% or more of leukemia cases are diagnosed in adults over 20 years of age. In particular, chronic lymphocytic leukemia (35%) and acute myeloid leukemia (AML) (32%) are the most common types of leukemia (Cancer Facts & Figures, Atlanta, American Cancer Society; 2014). According to the National Cancer Center Central Cancer Registry, 20.4% of AML patients are in their 70s or older and 18.4% thereof are in their 60s.

The revised guidelines for the initial diagnostic test items for hematological malignancies (In-Suk Kim et al., Lab Med Online 2020; 10(1): 10-24) recommend that a primary diagnostic test item for AML should be genetic mutation and the target genes be FLT3-ITD, NPM1, CEBPA, RUNX1, and KIT genes. In addition, the revised guidelines recommend that secondary test items should include FLT3-TKD, NRAS, TP53, ABL1 kinase, DNMT3A, IDH1, IDH2, TET2, MLL-PTD, ASXL1, ETV6, EZH2, CBL, and JAK2 gene mutations, and the test should be performed using the NGS panel.

Fms-Like Tyrosine kinase-3 (FLT3) is one of the most frequently mutated genes in acute myeloid leukemia (AML). Mutant FLT3 refers to a mutation expressed in leukemia cells that appears in a subpopulation of acute myeloid leukemia (AML) patients. Activating mutations in FLT3, such as internal tandem duplications (ITDs) in the juxtamembrane domain, occur in approximately 25-30% of newly diagnosed AML cases (Korean Patent No. 10-2018-0124055). Of these, overlapping FLT3-ITD mutations of 3 to 400 bases or more occur in approximately 25%, and point mutations in the tyrosine kinase domain occur in 7-10% (Tamara Castano-Bonilla et a., Scientific Reports. 2021 Oct volume 11, Article number: 20745, 2021).

Meanwhile, the determination of genetic mutation in acute myeloid leukemia (AML) is used as an indicator for diagnosis, detection of a minimal residual disease (MRD), and prediction of prognosis such as relapse or survival rate. Thereamong, FLT3 (fims-like tyrosine kinase-3) genetic mutation is the most common mutation in AML and it has been reported that the presence of FLT3 internal tandem duplication (FLT3-ITD) at the time of diagnosis leads to a poor prognosis and affects the relapse rate and survival rate. In addition, this induces the activity of tyrosine kinase. In addition, ITD of FLT3 varies greatly in length, and it has been reported that, as the length of ITD increases, symptoms become severe and prognosis becomes poorer (Kayser S et a., Blood. 2009 Sep 17;114(12):2386-92; Liu SB, et al., Haematologica. 2019 Jan;104(1):e9-e12).

Early FLT3-ITD mutation detection enables detection of some mutations in the ITD and TKD regions based on length differences using PCR and electrophoresis, but disadvantageously has a very low sensitivity of 10% and is incapable of accurately measuring mutation length and sequence. To overcome this, fragment analysis (FA) was developed to quantitatively analyze the amplified product after PCR (Korean Patent No. 10-2041001; LeukoStrat CDx FLT3 Mutation assay, Invivoscribe). Fragment analysis has advantages of having an improved sensitivity of about 3% compared to PCR and being capable of measuring the ITD length and ITD burden (ratio), but has disadvantages of being incapable of providing sequence analysis and not achieving the level of sensitivity (at least 10⁻⁴) required for MRD detection. To overcome these disadvantages, Invivoscribe, Inc. developed an AML-FLT3 ITD MRD Assay service using NGS, but has drawbacks of having a sensitivity of 5x10⁻⁵ and being incapable of simultaneously detecting mutations in the TKD region, which is important for MRD detection.

Meanwhile, various types of FLT3 inhibitors are used as drugs for AML. FLT3 inhibitors are largely divided into type I inhibitors and type II inhibitors. Type I inhibitors act in patients with FLT3-ITD or FLT3 kinase domain point mutations, and type II inhibitors act in patients with FLT-ITD mutations, but do not act in patients with FLT3 kinase domain point mutations. First-generation inhibitors include sunitinib, sorafenib, midostaurin, lestaurtinib, and tandutinib, which are known to be not specific for FLT3. On the other hand, second-generation inhibitors include quizartinib, crenolanib, and gilteritinib, which are known to be more specific for FLT3 (Larrosa-Garcia M, et al., Mol Cancer Ther. 2017 Jun;16(6):991-1001).

However, the FLT3 inhibitors have serious drug resistance when mutations occur or exist in the tyrosine kinase domain (TKD) region of the FLT3 gene. For example, it has been reported that drug resistance to type 1 inhibitors, gilteritinib and crenolanib, occurs when there is an F961L mutation in the FLT3 TKD1 region, and it has been reported that mutations in the D835 region of FLT3 TKD2 cause drug resistance to sunitinib, sorafenib, tandutinib, and quizartinib (Dilana Staudt et al., Int. J. Mol. Sic. 2018, 19, 3198).

Therefore, in order to comprehensively perform diagnosis, prognosis prediction, therapy strategy design, treatment, and monitoring of AML patients having FLT3-ITD mutations, technology for detecting mutations in the FLT3-ITD and TKD regions with high sensitivity is required.

Accordingly, as a result of great efforts to develop a composition that can be used for diagnosis, determination of presence of drug resistance, and prognosis prediction at high speed and in bulk of AML patients with FLT3-ITD mutations, the present inventors found that, when the FLT3 gene is amplified and analyzed using a primer set for amplifying the ITD and TKD regions of the FLT3 gene, diagnosis, drug resistance, and prognosis prediction of AML patients with FLT3-ITD mutations can be determined at high speed, with high sensitivity and accuracy. Based on this finding, the present invention has been completed.

### [Disclosure]

It is one object of the present invention to provide a composition for amplifying an FLT3 gene with improved sensitivity and accuracy.

It is another object of the present invention to provide a kit for amplifying an FLT3 gene containing the composition.

It is another object of the present invention to provide a method of diagnosing acute myeloid leukemia (AML) in a patient having an FLT3-ITD mutation using the composition.

It is another object of the present invention to provide a method for prescribing a targeted anticancer drug in an AML patient having an FLT3-ITD mutation using the composition.

It is another object of the present invention to provide a method of detecting minimal residual disease (MRD) in an acute myeloid leukemia (AML) patient having an FLT3-ITD mutation using the composition.

It is another object of the present invention to provide a method of predicting the prognosis of an acute myeloid leukemia (AML) patient having a FLT3-ITD mutation using the composition.

It is another object of the present invention to provide a method of determining resistance to a tyrosine kinase inhibitor, a therapeutic agent for acute myeloid leukemia (AML) in a patient having a FLT3-ITD mutation using the composition.

In accordance with one aspect of the present invention,
the above and other objects can be accomplished by the provision of a composition for amplifying a FLT3 gene containing the following primer sets:
(i) a first primer pair including a forward primer of SEQ ID NO. 1 and a reverse primer of SEQ ID NO. 2; (ii) a second primer pair including a forward primer of SEQ ID NO. 3 and a reverse primer of SEQ ID NO. 4; (iii) a seventh primer pair including a forward primer of SEQ ID NO. 13 and a reverse primer of SEQ ID NO. 14; (iv) an eighth primer pair including a forward primer of SEQ ID NO. 15 and a reverse primer of SEQ ID NO. 16; and (v) a ninth primer pair including a forward primer of SEQ ID NO. 17 and a reverse primer of SEQ ID NO. 188.

In accordance with another aspect of the present invention, provided is a composition for amplifying a FLT3 gene containing the following primer sets:
(i) a third primer pair including a forward primer of SEQ ID NO. 5 and a reverse primer of SEQ ID NO. 6; (ii) a fifth primer pair including a forward primer of SEQ ID NO. 9 and a reverse primer of SEQ ID NO. 10; (iii) a seventh primer pair including a forward primer of SEQ ID NO. 13 and a reverse primer of SEQ ID NO. 14; (iv) a ninth primer pair including a forward primer of SEQ ID NO. 17 and a reverse primer of SEQ ID NO. 18; (v) a fourth primer pair including a forward primer of SEQ ID NO. 7 and a reverse primer of SEQ ID NO. 8; (vi) a sixth primer pair including a forward primer of SEQ ID NO. 11 and a reverse primer of SEQ ID NO. 12; and (vii) an eighth primer pair including a forward primer of SEQ ID NO. 15 and a reverse primer of SEQ ID NO. 16.

In accordance with another aspect of the present invention, provided is a kit for amplifying an FLT3 gene containing the primer set.

In accordance with another aspect of the present invention, provided is a method of diagnosing acute myeloid leukemia (AML) in a patient having an FLT3-ITD mutation, or a method of providing information for diagnosing acute myeloid leukemia (AML) in the patient having the FLT3-ITD mutation, each method including: (a) extracting nucleic acids from a biological sample to obtain sequence information using the primer set; (b) aligning the sequence information (reads) to a reference genome database; (c) detecting an ITD mutation of FLT3 in the aligned sequence information (reads); and (d) determining that the patient has AML having an FLT3-ITD mutation when the ITD mutation of FLT3 is detected.

In accordance with another aspect of the present invention, provided is a method of treating acute myeloid leukemia (AML) in a patient having a FLT3-ITD mutation, or a method of providing information for treating acute myeloid leukemia (AML) in the patient with the FLT3-ITD mutation, the method including: (a) extracting nucleic acids from a biological sample to obtain sequence information using the primer set; (b) aligning the sequence information (reads) to a reference genome database; (c) detecting an ITD mutation of FLT3 in the aligned sequence information (reads); and (d) determining to prescribe a targeted anticancer agent when a mutation in the ITD region of FLT3 is detected.

In accordance with another aspect of the present invention, provided is a method of diagnosing acute myeloid leukemia (AML) in a patient having an FLT3-ITD mutation, or a method of providing information for diagnosing acute myeloid leukemia (AML) in the patient having the FLT3-ITD mutation, each method including: (a) extracting nucleic acids from a biological sample to obtain sequence information using the primer set; (b) aligning the sequence information (reads) to a reference genome database; (c) detecting an ITD mutation of FLT3 in the aligned sequence information (reads); and (d) determining that the patient has acute myeloid leukemia (AML) when the ITD mutation of FLT3 is detected.

In accordance with another aspect of the present invention, provided is a method of predicting the prognosis of an acute myeloid leukemia (AML) patient having a FLT3-ITD mutation, or a method of providing information for predicting the prognosis of the acute myeloid leukemia (AML) patient having the FLT3-ITD mutation, each method including: (a) extracting nucleic acids from a biological sample to obtain sequence information using the primer set; (b) aligning the sequence information (reads) to a reference genome database; (c) detecting an ITD mutation of FLT3 from the aligned sequence information (reads); and (d) predicting a prognosis based on the variant allele frequency (VAF) and the length of the detected ITD mutation of FLT3.

In accordance with another aspect of the present invention, provided is a method of determining the presence of resistance to a tyrosine kinase inhibitor, a therapeutic agent for acute myeloid leukemia (AML), in a patient having a FLT3-ITD mutation, or a method of providing information for determining the presence of resistance to a tyrosine kinase inhibitor, a therapeutic agent for acute myeloid leukemia (AML), in the patient having the FLT3-ITD mutation, each method including: (a) extracting nucleic acid from a biological sample to obtain sequence information using the primer set; (b) aligning the sequence information (reads) to a reference genome database; (c) detecting a TKD region mutation of FLT3 from the aligned sequence information (reads); and (d) detecting a TKD region mutation of FLT3 and determining a tyrosine inhibitor to which resistance is present depending on the type of the TKD region mutation.

In accordance with another aspect of the present invention, provided is use of the primer set for the preparation of an agent for diagnosing acute myeloid leukemia (AML) in a patient having an FLT3-ITD mutation.

In accordance with another aspect of the present invention, provided is use of the primer set for the preparation of an agent for detecting minimal residual disease (MRD) in an acute myeloid leukemia (AML) patient having an FLT3-ITD mutation.

In accordance with another aspect of the present invention, provided is use of the primer set for the preparation of an agent for predicting the prognosis of an acute myeloid leukemia (AML) patient having an FLT3-ITD mutation.

In accordance with another aspect of the present invention, provided is use of the primer set for the preparation of an agent for determining the presence of resistance to a tyrosine kinase inhibitor in a patient having an FLT3-ITD mutation.

### [Description of Drawings]

FIG. 1 is a schematic diagram illustrating regions of FLT3 genes amplified by primer sets according to the present invention.
FIG. 2 is a schematic diagram illustrating amplification regions of primer pairs constituting a first primer set capable of simultaneously amplifying ITD and TKD regions according to one embodiment of the present invention.
FIG. 3 is a schematic diagram illustrating amplification regions of primer pairs constituting a second primer set (A) and a third primer set (B) according to one embodiment of the present invention.
(A) of FIG. 4 is a schematic diagram illustrating a process for producing an amplicon using the first primer set according to the present invention, and (B) of FIG. 4 is a schematic diagram illustrating a process of producing an amplicon using both the second primer set and the third primer set.
(A) of FIG. 5 shows the linearity and sensitivity of the FLT3 mutation detected using the first primer set according to the present invention, and (B) of FIG. 5 shows the linearity and sensitivity of the FLT3 mutation detected using both the second primer set and the third primer set.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

As used herein, the term "NGS" means next generation sequencing or next generation base sequence analysis. NGS refers to a technology that includes fragmenting the whole genome and performing large-scale sequencing based on chemical reaction (hybridization) of the fragments or includes amplifying target gene regions using multiplex PCR and performing large-scale sequencing, and includes technologies from Agilent, Illumina, Roche, and Life Technologies. In a broad sense, NGS includes technologies from Pacific Biosciences, Nanopore Technology, etc., which are third-generation technologies, and fourth-generation technologies.

The technology originally referred to as "next generation sequencing (NGS)" corresponds to the second-generation technology in terms of automation. NGS is a name that is distinguished from the previous first automated device and the next NGS device (also referred to as the next-generation or third-generation NGS) that was formed later. However, as the development competition for efficient sequencing accelerates and sequencing technology based on the introduction of new technologies and the goal of platform use continues to be developed, the distinction between sequencing technologies of each generation becomes ambiguous. Therefore, NGS is used in a broad sense to encompass all sequencing technologies after automated Sanger sequencing.

The technologies introduced in NGS may be broadly divided into three types: clonal amplification, massively parallel sequencing, and base/color calling which is a new base sequence determination method (non-Sanger method) that can be read immediately. Clonal amplification has the effect of eliminating the cloning process by eliminating the library construction process, and massively parallel sequencing improves efficiency by handling hundreds of thousands of clones at the same time. The new base sequence determination method (non-Sanger method) that can be read immediately has the effect of eliminating the capillary electrophoresis process.

The process of obtaining template clones is simplified by clonal amplification. In order to perform Sanger sequencing, template DNA of about 500 base pairs in length is required. After constructing a BAC library, short fragments must be cloned by subcloning and then amplified in bacteria. The new method allows template clones to be obtained by directly cutting DNA into appropriately short fragments and amplifying the fragments using primers through PCR, without the cumbersome library construction and cloning processes. Strategies such as bead-based, solid-state, and DNA nanoball generation are used for clonal amplification.

Bead-based clonal amplification is performed using emulsion PCR. Emulsion PCR is a method in which a DNA library, which is a collection of fragmented genomic DNA, is spatially separated into small aqueous droplets in oil, and then amplified in an emulsion along with microbeads whose surfaces are modified with one PCR primer. This method allows at least 1 million clone DNA fragments derived from a single DNA fragment to be fixed to a single bead. A typical solid-state method is bridge-amplification. In the bridge-amplification, adapter oligonucleotides are ligated to both ends of fragmented DNA and are allowed to flow over the surfaces of glass flow cells so that they randomly bind to primers complementary to the adapters fixed to the surfaces thereof. When PCR is performed in this state, the free ends of the fixed DNA bind to free primers present in the vicinity to form a bridge and amplification proceeds. As amplification progresses, clusters that play the same role as the beads are formed.

NGS uses massively parallel sequencing to arrange the clones in the form of a sheet and perform sequencing. However, many template clones are required and separately preparing the template clones is time consuming. The process of reading the base sequence signal from the template also becomes a serious limiting factor that reduces efficiency. When hundreds of thousands of different clones are processed in a massively parallel manner, the time can be dramatically shortened.

In order to avoid the cumbersome electrophoresis process, a new method excluding the Sanger method, which includes reacting with the template and then reading the sequence information of each template directly from the signal generated during the reaction was developed. The base sequencing methods that replace the Sanger method are broadly divided into sequencing by DNA ligation (SBL) and sequencing by synthesis (SBS).

The SBL uses repetitive ligation of DNA fragments. More specifically, an anchor having n bases complementarily binds to the template DNA and a probe having two randomly encoded bases labeled with a fluorescent label and a degenerate or universal base following the same is added to the DNA library slide where the beads or clusters are precipitated. A probe having two encoded sequences complementary to the template DNA fragment immediately following the anchor is ligated to the anchor, and the two encoded base sequences are analyzed by fluorescent label imaging of the slide. Once the two sequences are analyzed, the degenerate base sequence and fluorescent particles are removed and then the process of adding probes is repeated. In addition to the n anchors, anchors having n+2 and n+4 bases are used and analyzed repeatedly to analyze the sequence of the entire template DNA fragment.

SBS is further divided into cyclic reversible termination (CRT) and single nucleotide addition (SNA).

CRT uses a process similar to the automated Sanger method and includes adding a mixture of primers, DNA polymerase, and modified nucleotides to a slide containing amplified DNA clusters using a solid-state method. The modified nucleotides are blocked with 3'-O-azidomethyl to prevent further polymerization and labeled with fluorescent labels which are specific to respective bases and are removable later. After polymerization, the unpolymerized bases are washed away and the bases are identified by imaging using a total internal reflection fluorescence (TIRF) microscope. Once the bases are identified, the fluorescent labels are decomposed and the 3'-OH is regenerated with a reducing agent, Tris(2-carboxyethyl)phosphine (TCEP). This process is repeated to analyze the sequence of the template DNA without electrophoresis.

The SNA is a method that analyzes the base sequence by converting the ions generated when DNA polymerase binds single nucleotides into light. The SNA is represented by the pyrosequencing method based on Roche's 454 instrument, which reads the pyrophosphate released when nucleotides are bound as light. This method includes repeating a process of sequentially reacting 4 types of dNTP (A, G, T, C) and removing the same. The base sequence is identified through light emitted whenever polymerization occurs.

A representative analysis device using SBL is the SOLiD series of the former Life Technologies, and a representative analysis device using SBS is the Hiseq and MiSeq series (CRT method) of Illumina, and the 454 series (SNA method) of Roche.

In the present invention, in order to diagnose AML patients and at the same time, determine if there is drug resistance, predict prognosis, and detect residual disease with high sensitivity and accuracy, a composition capable of accurately obtaining sufficient amounts of amplified products was designed.

That is, in one embodiment of the present invention, the ITD region and the TKD mutation region of the FLT3 gene were simultaneously amplified and analyzed using the NGS method. As a result, it was found that it is possible to detect the ITD of FLT3 with a length that could not detected by conventional methods with high sensitivity and accuracy, to identify the mutation in the TKD region and thus to simultaneously perform diagnosis of AML, determination of drug resistance, prediction of prognosis, and detection of residual disease (Tables 10 and 11).

Therefore, in one aspect, the present invention is directed to a composition for amplifying a FLT3 gene containing the following primer sets:
(i) a first primer pair including a forward primer of SEQ ID NO. 1 and a reverse primer of SEQ ID NO. 2;
(ii) a second primer pair including a forward primer of SEQ ID NO. 3 and a reverse primer of SEQ ID NO. 4;
(iii) a seventh primer pair including a forward primer of SEQ ID NO. 13 and a reverse primer of SEQ ID NO. 14;
(iv) an eighth primer pair including a forward primer of SEQ ID NO. 15 and a reverse primer of SEQ ID NO. 16; and
(v) a ninth primer pair including a forward primer of SEQ ID NO. 17 and a reverse primer of SEQ ID NO. 188.

In another aspect, the present invention is directed to a composition for amplifying a FLT3 gene containing the following primer sets:
(i) a third primer pair including a forward primer of SEQ ID NO. 5 and a reverse primer of SEQ ID NO. 6;
(ii) a fifth primer pair including a forward primer of SEQ ID NO. 9 and a reverse primer of SEQ ID NO. 10;
(iii) a seventh primer pair including a forward primer of SEQ ID NO. 13 and a reverse primer of SEQ ID NO. 14;
(iv) a ninth primer pair including a forward primer of SEQ ID NO. 17 and a reverse primer of SEQ ID NO. 18;
(v) a fourth primer pair including a forward primer of SEQ ID NO. 7 and a reverse primer of SEQ ID NO. 8;
(vi) a sixth primer pair including a forward primer of SEQ ID NO. 11 and a reverse primer of SEQ ID NO. 12; and
(vii) an eighth primer pair including a forward primer of SEQ ID NO. 15 and a reverse primer of SEQ ID NO. 16.

In another aspect, the present invention is directed to a method of diagnosing acute myeloid leukemia (AML) in a patient having an FLT3-ITD mutation, or a method of providing information for diagnosing acute myeloid leukemia (AML) in the patient having the FLT3-ITD mutation, each method including:
(a) extracting nucleic acids from a biological sample to obtain sequence information using the primer set;
(b) aligning the sequence information (reads) to a reference genome database;
(c) detecting an ITD mutation of FLT3 in the aligned sequence information (reads); and
(d) determining that the patient has AML having an FLT3-ITD mutation when the ITD mutation of FLT3 is detected.

In the present invention, step (a) may be performed by a method including the following steps:
(a-i) obtaining nucleic acids a biological sample;
(a-ii) removing proteins, fats, and other residues from the obtained nucleic acids using a salting-out method, a column chromatography method, or a bead method to obtain purified nucleic acids;
(a-iii) amplifying the purified nucleic acids or nucleic acids randomly fragmented by an enzymatic digestion, pulverization, or hydroshear method using the primer set described above and producing a single-end sequencing or paired-end sequencing library;
(a-iv) reacting the produced library with a next-generation sequencer; and
(a-v) obtaining sequence information (reads) of the nucleic acids in the next-generation sequencer.

In the present invention, the biological sample refers to any substance, biological fluid, tissue or cell obtained from or derived from a subject and may include, but is not limited to, whole blood, leukocytes, peripheral blood mononuclear cells, buffy coat, blood (including plasma and serum), bone marrow, and mixtures thereof.

In another aspect, the present invention is directed to a method of treating acute myeloid leukemia (AML) in a patient having a FLT3-ITD mutation, or a method of providing information for treating acute myeloid leukemia (AML) in the patient with the FLT3-ITD mutation, the method including:
(a) extracting nucleic acids from a biological sample to obtain sequence information using the primer set;
(b) aligning the sequence information (reads) to a reference genome database;
(c) detecting an ITD mutation of FLT3 in the aligned sequence information (reads); and
(d) determining to prescribe a targeted anticancer agent when a mutation in the ITD region of FLT3 is detected.

In the present invention, the targeted anticancer agent may be used without limitation so long as it is associated with a mutation in the FLT3-ITD region and preferably, any substance may be used without limitation so long as it inhibits the expression or decreases the activity of FLT3 to block or delay a biological pathway in which the FLT3 protein mediates, and preferably, the targeted anticancer agent includes at least one selected from the group consisting of small molecule compounds, antibodies or antigen-binding fragments thereof, aptamers, siRNA, shRNA, microRNA, and pharmaceutically acceptable salts thereof, but is not limited thereto.

In another aspect, the present invention is directed to a method of diagnosing minimal residual disease (MRD) in an acute myeloid leukemia (AML) patient having an FLT3-ITD mutation, or a method of providing information for diagnosing minimal residual disease (MRD) in the acute myeloid leukemia (AML) patient having the FLT3-ITD mutation, each method including:
(a) extracting nucleic acids from a biological sample to obtain sequence information using the primer set;
(b) aligning the sequence information (reads) to a reference genome database;
(c) detecting an ITD mutation of FLT3 in the aligned sequence information (reads); and
(d) determining that the patient has minimal residual disease (MRD) when the ITD mutation of FLT3 is detected.

As used herein, the term "minimal residual disease" (MRD) refers to a situation wherein, after a therapy for a tumor (e.g., chemotherapy, immunotherapy or targeted therapy), a morphologically normal sample (e.g., normal blood) may still contain a relative amount of residual malignant cells. Detection of minimal residual disease (MRD) is a new practical tool to more accurately measure remission induction during therapy. For a liquid tumor (e.g., lymphoma or myeloma), the term "MRD" may refer to a detection limit of 10⁻⁴ or less, for example, 10⁻⁵ or even 10⁻⁶ (Bettegowda et al., Sci Transl Med., 6(224), 224ra24, 2014).

In the present invention, the biological sample may be derived from a patient who is judged to have been completely treated of AML or a patient who undergoes anticancer treatment after being diagnosed with AML, but is not limited thereto.

In the present invention, the ITD may be the same as the ITD found at the time of AML diagnosis, but is not limited thereto.

It is known that, when a patient who was judged to have been completely cured receives treatment again in the stage wherein MRD increases even if AML has not relapsed, the response to treatment is better than after the progression (C. Rautenberg et al., Int. J. Mol. Sci. 2019, 20(1), 228.).

As used herein, the term "prognosis prediction" is used with the same meaning as "prognosis" and refers to an action of predicting the course and outcome of a disease in advance. More specifically, prognosis prediction may be interpreted as all actions of predicting the course of a disease after treatment by comprehensively considering the physiological or environmental condition of the patient, because the course of the disease after treatment vary depending on the physiological or environmental condition of the patient.

In terms of the objects of the present invention, the prognosis prediction may be interpreted as an action of predicting the course of the disease in advance after treatment of AML and predicting the risk of cancer progression, cancer recurrence and/or cancer metastasis. For example, the term "good prognosis" means that the risk of cancer progression, cancer recurrence and/or cancer metastasis of the patient after treatment of AML is lower than 1, which indicates that the AML patient has a high possibility of survival and is also expressed in another sense as a "positive prognosis". The term "bad prognosis" means that the risk of cancer progression, cancer recurrence and/or cancer metastasis of the patient after treatment of AML is higher than 1, which indicates that the AML patient has a high possibility of death and is also expressed in another sense as a "negative prognosis".

As used herein, the term "risk" refers to an odds ratio, a risk ratio, or the like for the probability that a patient will develop cancer progression, recurrence, and/or metastasis after treatment of AML.

In another aspect, the present invention is directed to a method of predicting the prognosis of an acute myeloid leukemia (AML) patient having a FLT3-ITD mutation, or a method of providing information for predicting the prognosis of an acute myeloid leukemia (AML) patient having the FLT3-ITD mutation, each method including:
(a) extracting nucleic acids from a biological sample to obtain sequence information using the primer set;
(b) aligning the sequence information (reads) to a reference genome database;
(c) detecting an ITD mutation of FLT3 from the aligned sequence information (reads); and
(d) predicting a prognosis based on the variant allele frequency (VAF) and the length of the detected ITD mutation of FLT3.

In the present invention, the prognosis prediction based on the ITD length and VAF may be used without limitation as long as it is a known value according to the correlation between the prognosis prediction of the patient and the length and VAF. It is generally obvious to those skilled in the art that, as the ITD length increases and the VAF becomes higher, the prognosis becomes bad.

For example, it is known that the prognosis is bad when the ITD length is at least 39 bp and at most 70 bp or more (Polak TB et al., Haematologica. 2022 Jul 7.).

In addition, in the prognosis prediction based on the ITD VAF, the European Leukemia Net (ELN) and the National Comprehensive Cancer Network (NCCN) set a reference value of the ITD VAF as 0.33.

Therefore, in the present invention, the prognosis prediction may be defined as follows: when the length of the ITD mutation is 39 bp or more and the VAF is 0.33 or more, the prognosis is defined as being the worst; when the length of the ITD mutation is 33 bp or more or the ITD VAF is 0.33 or more, the prognosis is defined as being bad; when the length of the ITD mutation is less than 33 bp or the ITD VAF is less than 0.33, the prognosis is defined as being moderate; and when the length of the ITD mutation is less than 33 bp and the ITD VAF is less than 0.33, the prognosis is defined as being the best; but is not limited thereto.

As used herein, the term "prognosis" means prediction of the progression of cancer, recurrence of cancer and/or the possibility of metastasis of cancer. The prediction method of the present invention may be used to make a decision on clinical treatment by selecting the most appropriate treatment method for any particular patient. The prediction method of the present invention is a valuable tool for diagnosis regarding the determination as to whether or not the progression of cancer, recurrence of cancer and/or the possibility of metastasis of cancer of a patient are likely to occur, and/or for assisting in such diagnosis.

In another aspect, the present invention is directed to a method of determining the presence of resistance to a tyrosine kinase inhibitor, a therapeutic agent for acute myeloid leukemia (AML), in a patient having a FLT3-ITD mutation, or a method of providing information for determining the presence of resistance to a tyrosine kinase inhibitor, a therapeutic agent for acute myeloid leukemia (AML), in the patient having the FLT3-ITD mutation, each method including:
(a) extracting nucleic acid from a biological sample to obtain sequence information using the primer set;
(b) aligning the sequence information (reads) to a reference genome database;
(c) detecting a TKD region mutation of FLT3 from the aligned sequence information (reads); and
(d) detecting a TKD region mutation of FLT3 and determining a tyrosine inhibitor to which resistance is present depending on the type of the TKD region mutation.

In the present invention, the tyrosine kinase inhibitor may be used without limitation as long as it is capable of treating AML and is preferably an FLT3 inhibitor, but is not limited thereto.

As used herein, the term "inhibitor" refers to a substance that inhibits the expression of FLT3 or reduces activity of FLT3, to block or delay a biological pathway in which the FLT3 protein mediates.

Any FLT3 inhibitor may be used as long as it interacts with the FLT3 and inhibits activity thereof and may include at least one selected from the group consisting of an antibody or an antigen-binding fragment thereof, an aptamer, siRNA, shRNA, microRNA, an inhibitory compound, and a pharmaceutically acceptable salt thereof, but is not limited thereto.

In the present invention, the FTL3 inhibitor may include at least one selected from the group consisting of sunitinib, lestaurtinib, sorafenib, quizartinib, midostaurin, pacritinib, gilteritinib, crenolanib, and tandutinib, but is not limited thereto.

In the present invention, the point mutation in the TKD region of the FLT3 gene may include the point mutations set in Table 2 below, but is not limited thereto.

**[Table 2]**

| Chr. | Start | End | Name | Exon |
|---|---|---|---|---|
| 13 | 28608085 | 28608087 | Sunitinib_A627P | 15 |
| 13 | 28608085 | 28608087 | Lestaurtinib_A627P | 15 |
| 13 | 28608085 | 28608087 | Sorafenib_A627P | 15 |
| 13 | 28608085 | 28608087 | Quizartinib_A627P | 15 |
| 13 | 28602340 | 28602342 | PKC412_(Midostaurin)_N676K | 16 |
| 13 | 28602352 | 28602354 | PKC412_(Midostaurin)_672E | 16 |
| 13 | 28602340 | 28602342 | PKC412_(Midostaurin)_N676D | 16 |
| 13 | 28602340 | 28602342 | PKC412_(Midostaurin)_N676I | 16 |
| 13 | 28602340 | 28602342 | PKC412_(Midostaurin)_N676S | 16 |
| 13 | 28601359 | 28601361 | PKC412_(Midostaurin)_F691L | 17 |
| 13 | 28601341 | 28601343 | PKC412_(Midostaurin)_G697R | 17 |
| 13 | 28601359 | 28601361 | PKC412_(Midostaurin)_F691I | 17 |
| 13 | 28601359 | 28601361 | Sunitinib_F691L | 17 |
| 13 | 28601359 | 28601361 | Sorafenib_F691L | 17 |
| 13 | 28601359 | 28601361 | Sorafenib_F691I | 17 |
| 13 | 28601359 | 28601361 | Quizartinib_F691L | 17 |
| 13 | 28601359 | 28601361 | Gilterinib_F691L | 17 |
| 13 | 28601359 | 28601361 | Crenolanib_F691L | 17 |
| 13 | 28592640 | 28592642 | Sunitinib_D835Y | 20 |
| 13 | 28592619 | 28592621 | Sunitinib_Y842C | 20 |
| 13 | 28592640 | 28592642 | Sorafenib_D835H | 20 |
| 13 | 28592640 | 28592642 | Sorafenib_D835V/F/I/del/Y/A | 20 |
| 13 | 28592619 | 28592621 | Sorafenib_Y842N | 20 |
| 13 | 28592619 | 28592621 | Sorafenib_Y842C | 20 |
| 13 | 28592640 | 28592642 | Tandutinib_D835Y | 20 |
| 13 | 28592640 | 28592642 | Quizartinib_D835V/F/I/del/Y | 20 |
| 13 | 28592640 | 28592642 | Quizartinib_D835H | 20 |
| 13 | 28592619 | 28592621 | Quizartinib_Y842C/H | 20 |
| 13 | 28592637 | 28592639 | type_II_FLT3_inhibitors_I836 | 20 |

In addition, it can be easily predicted by those skilled in the art that, since the composition of the present invention simultaneously amplifies the ITD region and the TKD mutation region of the FLT3 gene, it is possible to diagnose AML using the composition, to predict the prognosis based on the result of analysis if the sample is diagnosed as AML positive, and to select an appropriate treatment excluding drugs having drug resistance.

In addition, it can be easily predicted by those skilled in the art that, when the initial sample is AML positive, while the patient who supplied the sample is treated, the sample is continuously analyzed to detect MRD, when MRD is detected, whether or not there is drug resistance is determined using a combination of the MRD detection with the TKD mutation information of the patient, and a treatment plan can be established based on the determination result.

As used herein, the term "oligonucleotide" generally refers to a nucleotide polymer including about 10 to about 100 nucleotides. However, the length of the nucleotide may be 100 or more or 10 or less.

As used herein, the term "nucleotide" refers to a basic unit of nucleic acid containing a phosphate group, a 5-carbon sugar, and a nitrogen base. In RNA, the 5-carbon sugar is ribose. In DNA, the 5-carbon sugar is 2-deoxyribose. In a 5-nucleotide, the sugar contains a hydroxyl group (-OH) at the 5-carbon sugar-2. The term also includes analogs of the basic unit, such as a methoxy group at position 2 of ribose.

The primer of the present invention may be obtained in large quantities by cloning the desired sequence using a conventional cloning method (Maniatis, T., et al. Molecular Cloning: A Laboratory Manual, Cold Spring Harbor, New York, 1982) or performing chemical synthesis using a commercially available DNA synthesizer.

The primer of the present invention is preferably isolated or purified. The term "isolated or purified" means that a process for removing components other than the desired component from a natural or synthetic substance is used. The purity (percentage of the target primer contained in the total nucleic acid) of the isolated or purified primer at (w/w)% is generally 50% or more, preferably 70% or more, more preferably 90% or more, and most preferably 95% or more (e.g., 100%). The purity of the primer may be appropriately changed depending on the solvent and the state of the solid or liquid. The unit of purity may be (w/v)% or (v/v)%, and the desired purity may be appropriately calculated in consideration of the definition of purity (w/w)% mentioned above.

These primers may be provided as a solid in a dry state or an alcohol precipitate state, or may be provided as a solution in water or a suitable buffer (e.g., TE buffer, or the like).

As used herein, the term "amplicon" means an amplification product amplified by the primer.

In the present invention, the primer is capable of amplifying each gene, maintaining a GC ratio of 20 to 60%, and being complementary to a gene sequence having no tandem repeats.

As used herein, the term "amplification" refers to a reaction for amplifying a nucleic acid molecule. Various amplification reactions have been reported in the art, including, but being not limited thereto, polymerase chain reaction (hereinafter referred to as "PCR") (U.S. Pat. Nos. 4,683,195, 4,683,202, and 4,800,159), reverse transcription-polymerase chain reaction (hereinafter referred to as "RT-PCR") (Sambrook et al., Molecular Cloning. A Laboratory Manual, 3rd ed. Cold Spring Harbor Press (2001)), methods of WO 89/06700 and EP 329,822, ligase chain reaction (LCR, WO 90/01069), repair chain reaction (EP 439,182), transcription-mediated amplification (MA, WO 88/10315), self-sustained sequence replication (WO 90/06995), target polynucleotide selective amplification of target polynucleotide sequences (U.S. Patent No. 6,410,276), consensus sequence primed polymerase chain reaction (CP-PCR, U.S. Patent No. 4,437,975), arbitrarily primed polymerase chain reaction (AP-PCR, U.S. Patent Nos. 5,413,909 and 5,861,245), nucleic acid sequence-based amplification (NASSA, U.S. Patent Nos. 5,130,238, 5,409,818, 5,554,517, and 6,063,603), strand displacement amplification, and loop-mediated isothermal amplification (LAMP).

Other available amplification methods are disclosed in U.S. Pat. Nos. 5,242,794, 5,494,810, and 4,988,617 and U.S. Pat. No. 09/854,317.

PCR is the most well-known nucleic acid amplification method and many PCR variations and applications have been developed. For example, touchdown PCR, hot start PCR, nested PCR and booster PCR have been developed by modifying the traditional PCR procedure to enhance the specificity or sensitivity of PCR. In addition, real-time PCR, differential display PCR (D-PCR), rapid amplification of cDNA ends (RACE), DL-PCR (PC), inverse polymerase chain reaction (IPCR), vectorette PCR, and thermal asymmetric interlaced PCR (TAIL-PCR) have been developed for specific applications. More information on PCR is disclosed in McPherson, M.J., and Moller, S.G. PCR. BIOS Scientific Publishers, Springer-Verlag New York Berlin Heidelberg, N.Y. (2000), the teachings of which are incorporated herein by reference.

In the present invention, the primer set may include primer pairs represented by the combination set forth in Table 1 below.

**[Table 1]**

| Primer pair | SEQ ID NO | Name | Sequence (5' to 3') |
|---|---|---|---|
| First primer pair | 1 | ITD-E14-F | CGTGCATTTTAAAGATTTTCCAATGG |
| | 2 | ITD-E14-R | GAACTGCCTATTCCTAACTGACTC |
| Second primer pair | 3 | ITD-E15-F | TGCTGTCCTTCCACTATACTGTAC |
| | 4 | ITD-E15-R | GCCAAATGTTTCTGCAGCATTTCT |
| Third primer pair | 5 | 150(1)F | GGAAACTGTGCCTCCCATTT |
| | 6 | 150(1)R | GCTTTTGGAAAAGTGATGAACGC |
| Fourth primer pair | 7 | 150(2)F-1 | CCTGTTTTGCTAATTCCATAAGC |
| | 8 | 150(2)R | GATCTCAAATGGGAGTTTCCAA |
| Fifth primer pair | 9 | 150(3)F | CTGCAGAAACATTTGGCACA |
| | 10 | 150(3)R | GCCAGCTACAGATGGTACAGG |
| Sixth primer pair | 11 | 150(4)F | TCATTATCTGAGGAGCCGGT |
| | 12 | 150(4)R_n1 | CCTCTTCATTGTCGTTTTAACC |
| Seventh primer pair | 13 | TKD(16-2) -F | GGCACTCATGTCAGAACTCAAGA |
| | 14 | TKD(16-2) -R | AGGCATTCTTTATTTTGACCCCT |
| Eighth primer pair | 15 | TKD(17-1) -F | CAGAGAACCAAGCCCTCCTAAG |
| | 16 | TKD(17-1) -R | TGGATGTGATTGGAAAGTGGGG |
| Ninth primer pair | 17 | E20-TKD (5) -F | GGCACAGCCCAGTAAAGATA |
| | 18 | E20-TKD(5) -R | AAATAGCAGCCTCACATTGC |

In the present invention, DNA as a template for PCR may be prepared from a test sample. The test sample is not particularly limited and includes, for example, whole blood, leukocytes, peripheral blood mononuclear cells, buffy coat, blood (including plasma and serum), and bone marrow.

DNA may be prepared using a known method such as proteinase K/phenol extraction, phenol/chloroform extraction, alkaline dissolution, boiling, or the like. High-purity DNA may be prepared quickly and easily from a trace amount of sample using a commercially available DNA/RNA extraction kit.

The reaction conditions of PCR for gene amplification using the primer set of the present invention may be, for example, the following conditions:
Heat denaturation (e.g., 92°C to 98°C)
Annealing (e.g., 55°C to 72°C)
Extension (e.g., 65°C to 80°C)

In the PCR mentioned above, the annealing and the extension may be performed in one step (shuttle method) or using a method including setting the annealing temperature to a higher level and gradually lowering the temperature in each cycle (touchdown method). Alternatively, these methods may be combined. When the shuttle method is performed, the temperature of the annealing and the extension is typically 65 to 72°C.

In the PCR mentioned above, the annealing and the extension may be performed in one step (shuttle method), or using a method including setting the annealing temperature to a higher level and gradually lowering the temperature in each cycle (touchdown method). Alternatively, these methods may be combined. When the shuttle method is performed, the temperature of the annealing and the extension is typically 65 to 72°C.

The method of sequence analysis using NGS varies depending on the type of NGS and is performed according to the manual of the corresponding manufacturer (e.g., NexteraR XT DNA Library Prep Reference Guide). Paired-end analysis is preferably used to sequence the obtained sample. Then, a summary of the sequence analysis procedure using MiSeq from Illumina, Inc. is described. Although other devices (such as Ion Proton produced by Life Technologies, GS FLX+ from Roche Diagnostics K.K.) are used, the base sequence may be determined similarly using a method suitable for the device.
1. The amplified product of each sample is subjected to tagmentation using a kit (e.g., Nextera XT DNA Sample Preparation Kit (Illumina, Inc.)).
2. Using a kit (e.g., Nextera XT v2 Index Kit Set A, B, C, D (Illumina, Inc.)), different index sequences are added to both sides of the obtained fragment sequences for each sample and PCR is performed.
3. The amplified product is purified.
4. The library size is confirmed using the amplified product from each sample.
5. The concentration between samples is adjusted.
6. The amplified product of the sample is pooled and the pooled amplified product is subjected to quantitative PCR.
7. The pooled amplified product is sequenced using MiSeq.

In this way, whether or not the gene is mutated may be determined using a database (hereinafter referred to as "read") based on the base sequence information of the amplified product obtained by NGS.

In another aspect, the present invention is directed to a kit for amplifying the FLT3 gene including the primer set.

The kit of the present invention may further include other reagents required for PCR in addition to the primer set of the present invention. When the reagent does not adversely affect the reaction after being preserved in combination with the primer set of the present invention, it may be mixed with the primer set and incorporated into the kit. Alternatively, the reagent and the primer set of the present invention may be provided separately rather than being mixed. Examples of the reagent include a DNA extraction reagent, a DNA polymerase enzyme, dNTP, a reaction buffer, a DNA molecule including a target sequence that serves as a positive control in PCR, a manual, and the like. The DNA polymerase enzyme may be a commercially available product.

In another aspect, the present invention is directed to the use of the primer set for the preparation of an agent for diagnosing acute myeloid leukemia (AML) in a patient having an FLT3-ITD mutation.

In the present invention, the agent for diagnosing AML may be used in a method of diagnosing AML, including: (a) extracting nucleic acids from a biological sample to obtain sequence information using the diagnostic agent; (b) aligning the sequence information (reads) to a reference genome database; (c) detecting an ITD mutation of FLT3 in the aligned sequence information (reads); and (d) determining whether or not the patient has AML having an FLT3-ITD mutation when the ITD mutation of FLT3 is detected, but is not limited thereto.

In another aspect, the present invention is directed to the use of the primer set for the preparation of an agent for treating an acute myeloid leukemia (AML) patient having an FLT3-ITD mutation.

In another aspect, the present invention is directed to the use of the primer set for the preparation of an agent for detecting minimal residual disease (MRD) in an acute myeloid leukemia (AML) patient having an FLT3-ITD mutation.

In the present invention, the agent for detecting minimal residual disease (MRD) may be used in a method of detecting minimal residual disease (MRD) in an acute myeloid leukemia (AML) patient having an FLT3-ITD mutation, including: (a) extracting nucleic acids from a biological sample to obtain sequence information using the diagnostic agent; (b) aligning the sequence information (reads) to a reference genome database; (c) detecting an ITD mutation of FLT3 in the aligned sequence information (reads); and (d) determining whether or not the patient has minimal residual disease (MRD) when the ITD mutation of FLT3 is detected, but is not limited thereto.

In another aspect, the present invention is directed to the use of the primer set for the preparation of an agent for predicting the prognosis of an acute myeloid leukemia (AML) patient having an FLT3-ITD mutation.

In the present invention, the agent for predicting the prognosis may be used in a method of predicting the prognosis of an acute myeloid leukemia (AML) patient having an FLT3-ITD mutation, including: (a) extracting nucleic acids from a biological sample to obtain sequence information using the primer set; (b) aligning the sequence information (reads) to a reference genome database; (c) detecting an ITD mutation of FLT3 from the aligned sequence information (reads); and (d) predicting a prognosis based on the variant allele frequency (VAF) and the length of the detected ITD mutation of FLT3, but is not limited thereto.

In another aspect, the present invention is directed to the use of the primer set for the preparation of an agent for determining the presence of resistance to a tyrosine kinase inhibitor in a patient having an FLT3-ITD mutation.

In the present invention, the agent for determining the presence of resistance may be used in a method of determining the presence of resistance to a tyrosine kinase inhibitor, a therapeutic agent for acute myeloid leukemia (AML), in a patient having an FLT3-ITD mutation including: (a) extracting nucleic acid from a biological sample to obtain sequence information using the primer set; (b) aligning the sequence information (reads) to a reference genome database; (c) detecting a TKD region mutation of FLT3 from the aligned sequence information (reads); and (d) detecting a TKD region mutation of FLT3 and determining a tyrosine inhibitor to which resistance is present depending on the type of the TKD region mutation.

Hereinafter, the present invention will be described in more detail with reference to examples. However, it will be obvious to those skilled in the art that these examples are provided only for illustration of the present invention, and should not be construed as limiting the scope of the present invention.

### Example 1. Production of primers for analysis of biological samples

In order to effectively analyze the ITD length and mutation of the FTL3 gene, primers of SEQ ID NOS. 1 to 18 having the same configuration as in Table 1 were designed, and then primers shown in Table 3 below in which an Illumina Nextera adapter sequence is attached to the 5' end of each primer to improve the subsequent operation speed were produced.

**[Table 3]**

| Primer pair | SEQ ID NO | Name | Sequence (5' to 3') |
|---|---|---|---|
| First primer pair_P | 19 | ITD-E14-F_P | TCGTCGGCAGCGTCAGATGTGTATAAGAGACAGCG TGCATTTTAAAGATTTTCCAATGG |
| | 20 | ITD-E14-R_P | GTCTCGTGGGCTCGGAGATGTGTATAAGAGACAGG AACTGCCTATTCCTAACTGACTC |
| Second primer pair_P | 21 | ITD-E15-F_P | TCGTCGGCAGCGTCAGATGTGTATAAGAGACAGTG CTGTCCTTCCACTATACTGTAC |
| | 22 | ITD-E15-R_P | GTCTCGTGGGCTCGGAGATGTGTATAAGAGACAGG CCAAATGTTTCTGCAGCATTTCT |
| Third primer pair_P | 23 | 150(1)F_P | TCGTCGGCAGCGTCAGATGTGTATAAGAGACAGGG AAACTGTGCCTCCCATTT |
| | 24 | 150(1)R_P | GTCTCGTGGGCTCGGAGATGTGTATAAGAGACAGG CTTTTGGAAAAGTGATGAACGC |
| Fourth primer pair_P | 25 | 150(2)F-1_P | TCGTCGGCAGCGTCAGATGTGTATAAGAGACAGCC TGTTTTGCTAATTCCATAAGC |
| | 26 | 150(2)R_P | GTCTCGTGGGCTCGGAGATGTGTATAAGAGACAGG ATCTCAAATGGGAGTTTCCAA |
| Fifth primer pair_P | 27 | 150(3)F_P | TCGTCGGCAGCGTCAGATGTGTATAAGAGACAGCT GCAGAAACATTTGGCACA |
| | 28 | 150(3)R_P | GTCTCGTGGGCTCGGAGATGTGTATAAGAGACAGG CCAGCTACAGATGGTACAGG |
| Sixth primer pair_P | 29 | 150(4)F_P | TCGTCGGCAGCGTCAGATGTGTATAAGAGACAGTC ATTATCTGAGGAGCCGGT |
| | 30 | 150(4)R-n1_P | GTCTCGTGGGCTCGGAGATGTGTATAAGAGACAGC CTCTTCATTGTCGTTTTAACC |
| Seventh primer pair_P | 31 | TKD(16-2) - F_P | TCGTCGGCAGCGTCAGATGTGTATAAGAGACAGGG CACTCATGTCAGAACTCAAGA |
| | 32 | TKD(16-2) - R_P | GTCTCGTGGGCTCGGAGATGTGTATAAGAGACAGA GGCATTCTTTATTTTGACCCCT |
| Eighth primer pair_P | 33 | TKD(17-1) - F_P | TCGTCGGCAGCGTCAGATGTGTATAAGAGACAGCA GAGAACCAAGCCCTCCTAAG |
| | 34 | TKD(17-1) - R_P | GTCTCGTGGGCTCGGAGATGTGTATAAGAGACAGT GGATGTGATTGGAAAGTGGGG |
| Ninth primer pair_P | 35 | E20-TKD(5)-F_P | TCGTCGGCAGCGTCAGATGTGTATAAGAGACAGGG CACAGCCCAGTAAAGATA |
| | 36 | E20-TKD(5)-R_P | GTCTCGTGGGCTCGGAGATGTGTATAAGAGACAGA AATAGCAGCCTCACATTGC |

| | | | |
|---|---|---|---|
| * Underlined sequence: Nextera adaptor sequence | | | |

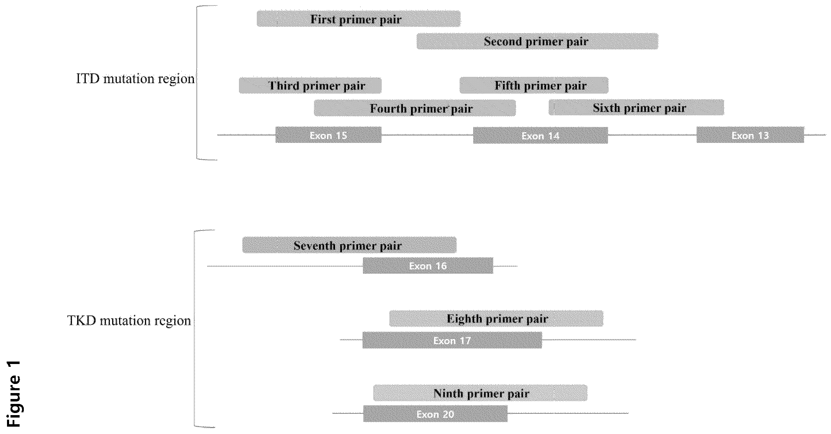

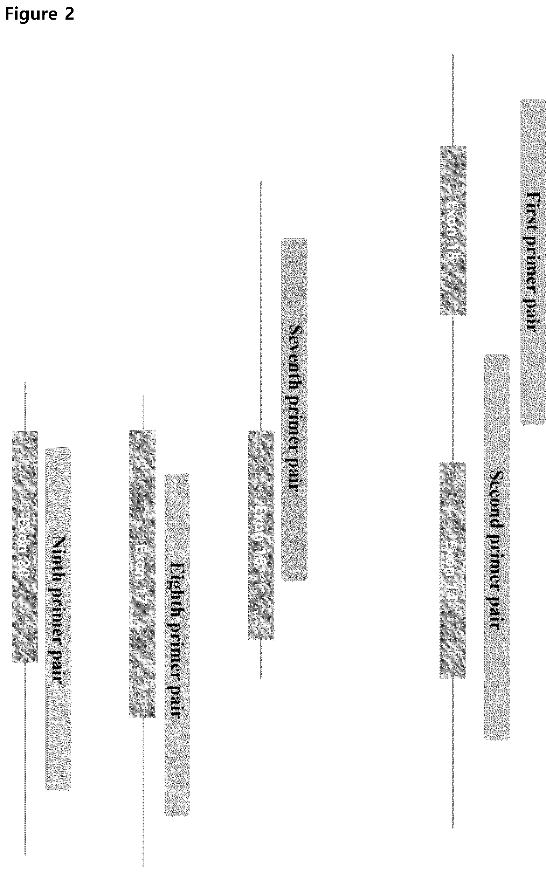

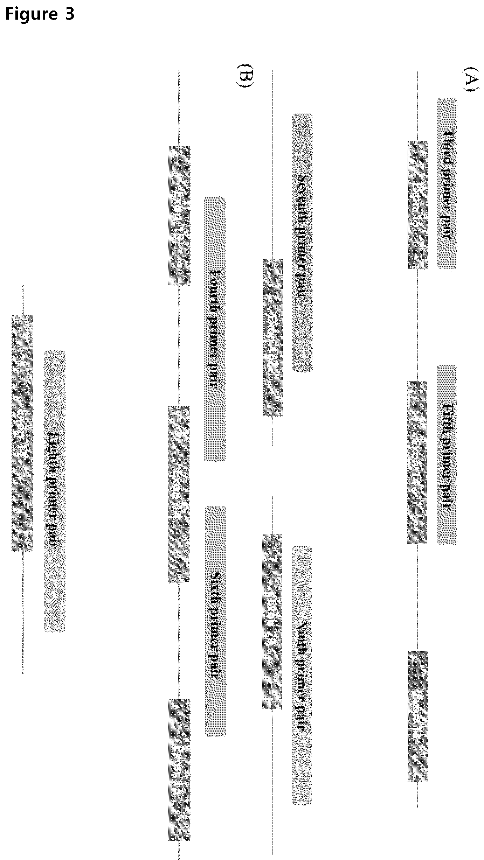

In addition, as shown in FIGS. 1 to 3, each primer pair has different amplification regions across the ITD region and the TKD region. The primer set capable of efficiently amplifying these regions was produced as shown in Table 4 below.

**[Table 4]**

| Primer set | Primer pair | SEQ ID NO | |
|---|---|---|---|
| | | Forward primer | Reverse primer |
| First primer set | First primer pair | 1 | 2 |
| | Second primer pair | 3 | 4 |
| | Seventh primer pair | 13 | 14 |
| | Eighth primer pair | 15 | 16 |
| | Ninth primer pair | 17 | 18 |
| Second primer set | Third primer pair | 5 | 6 |
| | Fifth primer pair | 9 | 10 |
| | Seventh primer pair | 13 | 14 |
| Third primer set | Fourth primer pair | 7 | 8 |
| | Sixth primer pair | 11 | 12 |
| | Eighth primer pair | 15 | 16 |
| | Ninth primer pair | 17 | 18 |

### Example 2. Amplicon amplification and library construction of ITD and TKD regions of FLT3 gene using produced primers

### 2-1. Amplicon amplification and library construction using first primer set

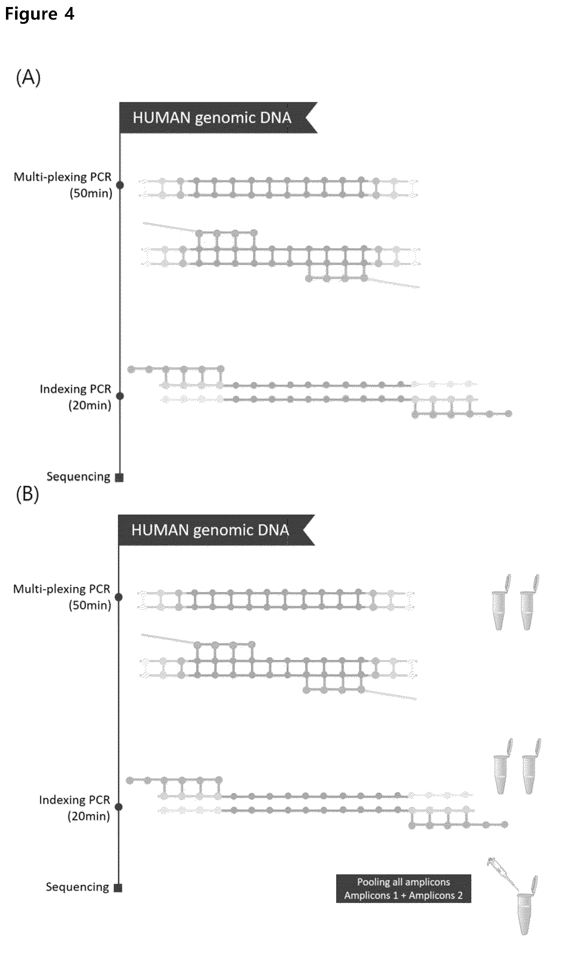

Amplicons were constructed from genomic DNA isolated from cell lines (MV-4-11, ATCC, CRL-9591), standard materials (HD829 and HD734 Horizon Discovery and Myeloid Mutation DNA Mix, SeraCare, 0710-0408), and clinical samples obtained from AML patients using the same method as in (A) of FIG. 4.

That is, the reaction products in Table 5 were subjected to primary amplification under the reaction conditions in Table 6, and then the reaction products in Table 7 were subjected to indexing-PCR under the conditions in Table 8 to produce the final amplicons and thereby construct the library. The reagent used for the primary amplification reaction herein was KOD -Multi & Epi-^{™} (TOYOBO, KME-101), and the reagents used for the secondary amplification reaction herein were NEBNext^{®} Ultra^{™} II Q5^{®} Master Mix (New England BioLabs, M0544) and IDT^{®} for Illumina^{®} DNA/RNA UD Indexes (Illumina, 20026121).

**[Table 5]**

| Components | x1 (µL) |
|---|---|
| First primer set | 5 |
| Polymerase | 2 |
| 2X Buffer | 25 |
| Template | Up to 18 |
| D. W. | 18 - template vol. |
| **Total** | **50** |

**[Table 6]**

| **Temp.** | **Time** | **Cycle** |
|---|---|---|
| 94°C | 2 min | 1 |
| 98°C | 10 sec | 20 |
| 61°C | 30 sec | |
| 68°C | 30 sec | |
| 4°C | Hold | - |

**[Table 7]**

| Components | x1 (µL) |
|---|---|
| Polymerase mix | 25 |
| UDI Dual index (i7+i5) | 5 |
| Eluate from first step | 15 |
| D.W. | 5 |
| **Total** | **50** |

**[Table 8]**

| **Temp.** | **Time** | **Cycle** |
|---|---|---|
| 98°C | 30 sec | 1 |
| 98°C | 10 sec | 8 |
| 61°C | 75 sec | |
| 65°C | 5 min | 1 |
| 4°C | hold | - |

### 2-2. Amplicon amplification and library production using second primer set and third primer set

When primary amplification was performed using the second primer set and the third primer set as shown in B of FIG. 4 using the same sample, the target region was amplified independently for each reaction product in Table 5 under the reaction conditions of Table 6, and then each reaction product in Table 9 was subjected to indexing-PCR under the conditions of Table 8 to produce the final amplicon and thereby produce a library.

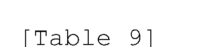

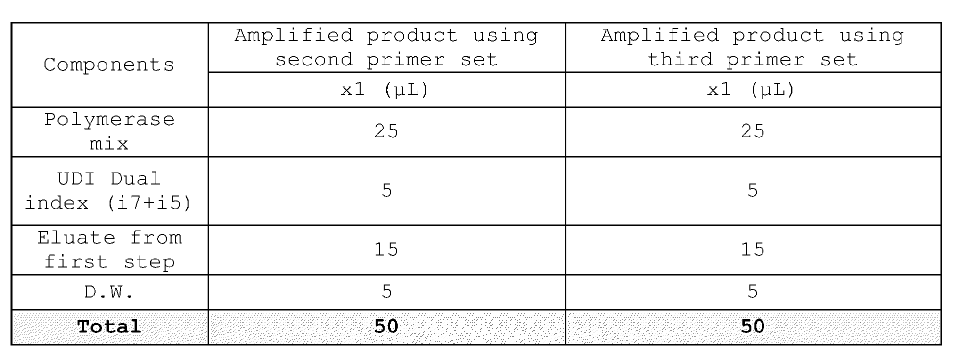

### Example 3. Verification of accuracy of detection of ITD mutation and TKD mutation of FLT3 gene using produced primers

The library produced using the method of Example 2 was sequenced using MiSeq or MiSeqDx equipment to produce reads, and then the accuracy of mutation detection in the corresponding region in each sample was verified.

**[Table 10]**

| Results of ITD mutation detection using first primer set and second and third primer sets | | | | | | |
|---|---|---|---|---|---|---|
| Sample type | Sampl e name | Mut. type | *Length, VAF | First primer set result | Second and third primer sets result | FA analysis result |
| Cell line | MV-4-11 | ITD | LOH, 30 bp, 100% | **30 bp, 97.5%** | **30 bp, 95.5%** | 31 bp, 100% |
| Standa rd | SeraC are (0710-0408) | ITD | 33 bp, 10% | **33 bp, 7.8%** | **33 bp, 12.4%** | 35 bp, 7.2% |
| | | | 42 bp, 5% | **42 bp, 4.6%** | **42 bp, 8.5%** | 44 bp, 3.7% |
| Clinical specimen #1 | | ITD | 6 bp, 26.9% | **6 bp, 25.4%** | **6 bp, 27.2%** | 7 bp, 31.7% |
| Clinical specimen #2 | ITD | 34 bp, 2.1% | **34 bp, 4.0%** | **34 bp, 2.5%** | 38 bp, 4.2% | |

| | | | | | | |
|---|---|---|---|---|---|---|
| * Cell line, standard material length, and VAF results are provided by the manufacturer/Clinical sample results are obtained using diagnostic product of the present inventor (HEMEaccuTest^{™} DNA) using NGS-hyb | | | | | | |

**[Table 11]**

| Results of ITD mutation detection using first primer set, and second and third primer sets | | | | | | |
|---|---|---|---|---|---|---|
| Sample type | Sampl e name | Mut. type | *Length, VAF | First primer set result | Second and third primer sets result | FA analysis result |
| Standa rd | HD829 | TKD | D835Y, 5% | **D835Y, 4.8%** | **D835Y, 4.9%** | D835Y, 4.0% |
| Standa rd | SeraC are (0710-0408) | TKD | D835Y, 10% | **D835Y, 11.4%** | **D835Y, 12.1%** | D835Y, 20.0% |
| Clinical specimen #1 | | TKD | D835Y, 20% | **D835Y, 20.5%** | **D835Y, 20.4%** | D835Y, 32.6% |
| Clinical specimen #3 | | TKD | D835E, 49.8% | **D835E, 51.6%** | **D835E, 51.2%** | D835E, 53.8% |

| | | | | | | |
|---|---|---|---|---|---|---|
| * Cell line, standard material length, and VAF results are provided by the manufacturer/Clinical sample results are obtained using diagnostic product of the present inventor (HEMEaccuTest^{™} DNA) using NGS-hyb | | | | | | |

As a result, as can be seen from Table 10, the ITD mutation of the FLT3 gene could be detected similar to the conventional method by using the first primer set or the second/third primer set

In addition, as can be seen from Table 11, the TKD mutation of the FLT3 gene could be detected similar to the conventional method using the first primer set or the second/third primer set of the present invention.

### Example 4. Comparison between method of detecting ITD mutation and TKD mutation of FLT3 genes using produced primers and fragment analysis

Fragment analysis is a conventional method that is the most widely used in FLT3-ITD mutation detection and has the lowest sensitivity. Fragment analysis is known to have a sensitivity of about 3%. The library was produced using the method of Example 2 from standard materials and clinical samples in which ITD and TKD mutations were not detected by the directly performed fragment analysis (FLT3 Mutation Assay, Invivoscribe, 14120031), and sequenced using MiSeq or MiSeqDx equipment to produce reads. Then, mutations in the corresponding region in each sample were detected and the results were compared with the results of the fragment analysis.

**[Table 12]**

| # | Sample Type | Sampl e | Fragment analysis result | Details of NGS results | | | Remarks |
|---|---|---|---|---|---|---|---|
| | | | | Detectio n ITD length | First primer set result | Second and third primer set results | |
| 1 | Standar d | HD829 | Undetecte d | 300 bp | Detecte d | Detecte d | Information provided by manufacture r: 300 bp, 5% |
| 2 | Clinical specimen #4 | | Undetecte d | 54 bp | Detecte d | Detecte d | |
| 3 | Clinical specimen #5 | | Undetecte d | 54 bp | Detecte d | Detecte d | |
| | | | Undetecte d | 106 bp | Detecte d | Detecte d | |
| 4 | Clinical specimen #6 | | Undetecte d | 156 bp | Detecte d | Detecte d | |
| 5 | Clinical specimen #7 | | Undetecte d | 156 bp | Detecte d | Detecte d | |
| 6 | Clinical specimen #8 | | Undetecte d | 48 bp | Detecte d | Detecte d | |
| 7 | Clinical specimen #9 | | Undetecte d | 48 bp | Detecte d | Detecte d | |
| 8 | Clinical specimen #10 | | Undetecte d | 48 bp | Detecte d | Detecte d | |
| 9 | Clinical specimen #11 | | Undetecte d | 78 bp | Detecte d | Detecte d | |

**[Table 13]**

| # | Sample type | Sampl e | Fragment analysis result | NGS result | Details of NGS results | | | Remar ks |
|---|---|---|---|---|---|---|---|---|
| | | | | | Detecti on TKD positio n | First prime r set resul t | Second and third primer set result | |
| 1 | Standa rd | HD734 | Undetect ed | Detect ed | D835Y | 1.3% | 1.2% | Infor matio n provi ded by manuf actur er: D835y, 1.3% |
| | | | Undetect ed | Detect ed | ΔI836 | 1.2% | 1.2% | Infor matio n provi ded by manuf actur er: Δ I836, 1.3% |
| 2 | Clinical specimen #12 | | Undetect ed | Detect ed | D835Y | 1.1% | 1.1% | |
| | | | Undetect ed | Detect ed | N676K | 19.95 % | 20.2% | |

As a result, as can be seen from Tables 12 and 13, ITD mutations that were not detected in standard materials and clinical samples due to low sensitivity of fragment analysis were detected as positive in the NGS results.

In particular, the HD829 standard material #1 in Table 12 has a 300 bp ITD of about 5%. Theoretically, fragment analysis has a detectable ITD length of 270 bp and a sensitivity of about 3%. The non-detection result is considered because an ITD length is out of a detectable range.

In addition, as can be seen from Table 13, TKD mutations that were not detected in the standard material and clinical samples due to the low sensitivity of fragment analysis were detected as positive in the NGS results.

In particular, clinical sample #2 in Table 13 has a TKD mutation at amino acid 676, but only TKD mutations at positions 835 and 836 could be detected by existing fragment analysis. The non-detection result is considered because a TKD position is out of a detectable range.

### Example 5. Comparison between method of detecting ITD mutation and TKD mutation of FLT3 gene using produced primer and conventional products and methods

The library produced using the method of Example 2 was sequenced using MiSeq or MiSeqDx to produce reads, and then mutations in the corresponding region in each sample were detected and compared with those of conventional products and technologies.

**[Table 14]**

| **#** | **Item** | **Prese nt inven tion** | **NGS** | **Capillary Electrophoresis** (Fragment Analysis) | **Sang er Sequ enci ng** | **PCR(Gel) / Electrophores is** |
|---|---|---|---|---|---|---|
| 1 | Represen tative Products and Services | -- | -Invivoscribe, Inc. | | No prod uct | -Inivoscribe, Inc. |
| | | | | - Inivoscribe 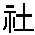 | | - LeukoStrat^{®} **CDx*** FLT3 Mutation Assay |
| | | | | - LeukoStrat^{®} **CDx*** FLT3 Mutation Assay | | |
| | | | | - IVD | | - IVD |
| | | | - amplicon-based NGS | | | |
| | | | | | | -Invivoscribe, Inc. |
| | | | -LDT(Laborator y developed test) service | - Invivoscribe 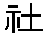 | | |
| | | | | - FLT3 Mutation Assay for ABI Fluorescence Detection | | |
| | | | | | | - FLT3 Mutation Assay for ABI Fluorescence Detection |
| | | | | - RUO | | |
| | | | | | | - RUO |
| 2 | Sensitiv ity | 10⁻⁵ (0.00 1%) | 5 x 10⁻⁵ (0.005%) | 3% | 20% | 10% |
| 3 | Detectab le ITD length | ~300b p | ~126bp | 271±1bp | ~700 bp | >14bp (*detection of short length is difficult) |
| 4 | TKD mutation detectio n | Detec tion of all TKD mutat ions is possi ble | Impossible | Detection of only D835 and 1836 is possible | Dete ctio n of all TKD muta tion s is poss ible | Impossible |

As a result, as can be seen from Table 14, the method of the present invention provides a detectable ITD length more than twice that of the conventional NGS method and is capable of detecting all TKD mutations and enabling processing of more samples in a shorter time than in Sanger sequencing.

### Example 6. Linearity of method of detecting ITD mutation and TKD mutation of FLT3 genes using produced primers

The library produced using the method of Example 2 was sequenced using MiSeq or MiSeqDx to produce reads, then clinical specimen #3 including a 97 bp ITD mutation was serially diluted to detect mutations in the corresponding region in each sample, and then linearity was analyzed.

A library of #3 clinical sample was produced using the method of Example 2, reads were produced using MiSeq or MiSeqDx, ITD mutations were detected, and then mutation rates (Allelic Fraction) were confirmed through a bioinformatics analysis pipeline.

The Pindel program was used to detect ITD mutations. This program is one of the most widely used programs for detecting FLT3-ITD mutations using NGS data and is characterized in that it can provide the length, sequence information, occurrence location, and mutation rate of ITD mutations.

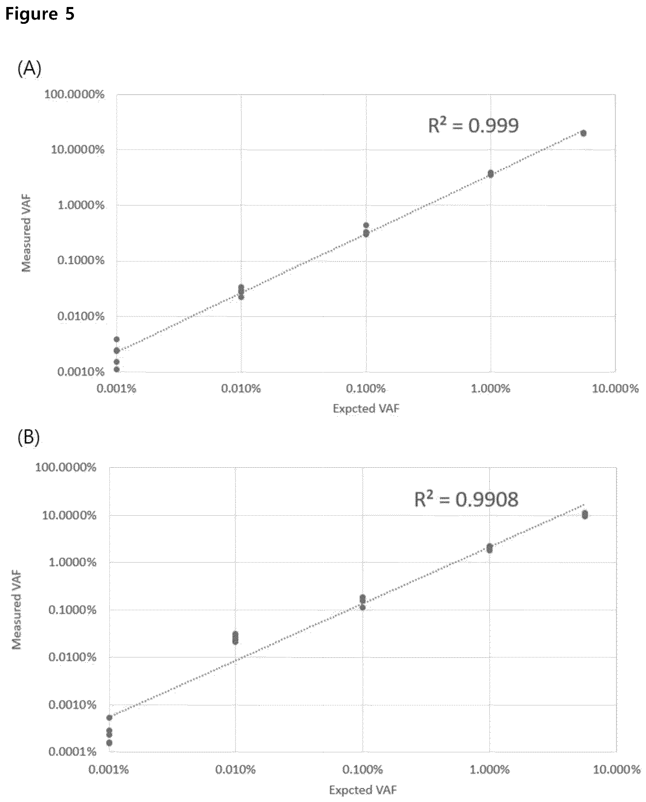

As a result, as can be seen from FIG. 5, the predicted and measured values were directly proportional, with a high linearity of R²=0.99. In addition, mutations were detected at a lower limit measurement interval of 0.001% and mutation detection was found at a minimum detection limit (based on expected VAF).

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Industrial applicability]

The composition for amplifying genes according to the present invention is capable of diagnosing acute myeloid leukemia (AML) in a patient having an FLT3-ITD mutation, determining prescription of targeted anticancer therapy in an AML patient having an FLT3-ITD mutation, detecting minimal residual disease (MRD) in an AML patient, predicting the prognosis of an AML patient; and determining whether there is drug resistance to an AML tyrosine kinase inhibitor, thereby shortening the time for obtaining results of analysis from samples and enabling efficient testing, which is useful because it allows for selection of correct and rapid diagnostic and therapeutic methods for acute myeloid leukemia patients and thus enables early treatment and relapse prevention.

### [Sequence Listing Free Text]

An electronic file is attached.

## Claims

1. A composition for amplifying an FLT3 gene comprising the following primer sets:
(i) a first primer pair including a forward primer of SEQ ID NO. 1 and a reverse primer of SEQ ID NO. 2;
(ii) a second primer pair including a forward primer of SEQ ID NO. 3 and a reverse primer of SEQ ID NO. 4;
(iii) a seventh primer pair including a forward primer of SEQ ID NO. 13 and a reverse primer of SEQ ID NO. 14;
(iv) an eighth primer pair including a forward primer of SEQ ID NO. 15 and a reverse primer of SEQ ID NO. 16; and
(v) a ninth primer pair including a forward primer of SEQ ID NO. 17 and a reverse primer of SEQ ID NO. 188.

2. A composition for amplifying an FLT3 gene comprising the following primer sets:
(i) a third primer pair including a forward primer of SEQ ID NO. 5 and a reverse primer of SEQ ID NO. 6;
(ii) a fifth primer pair including a forward primer of SEQ ID NO. 9 and a reverse primer of SEQ ID NO. 10;
(iii) a seventh primer pair including a forward primer of SEQ ID NO. 13 and a reverse primer of SEQ ID NO. 14;
(iv) a ninth primer pair including a forward primer of SEQ ID NO. 17 and a reverse primer of SEQ ID NO. 18;
(v) a fourth primer pair including a forward primer of SEQ ID NO. 7 and a reverse primer of SEQ ID NO. 8;
(vi) a sixth primer pair including a forward primer of SEQ ID NO. 11 and a reverse primer of SEQ ID NO. 12; and
(vii) an eighth primer pair including a forward primer of SEQ ID NO. 15 and a reverse primer of SEQ ID NO. 16.

3. A kit for amplifying an FTL3 gene comprising the primer set according to claim 1 or 2.

4. A method of diagnosing acute myeloid leukemia (AML) in a patient having an FLT3-ITD mutation, the method comprising:
(a) extracting nucleic acids from a biological sample to obtain sequence information using the primer set according to claim 1 or 2;
(b) aligning the sequence information (reads) to a reference genome database;
(c) detecting an internal tandem duplication (ITD) mutation of FLT3 in the aligned sequence information (reads); and
(d) determining that the patient has AML when the ITD mutation of FLT3 is detected.

5. A method of treating acute myeloid leukemia (AML) in a patient having an FLT3-ITD mutation, the method comprising:
(a) extracting nucleic acids from a biological sample to obtain sequence information using the primer set according to claim 1 or 2;
(b) aligning the sequence information (reads) to a reference genome database;
(c) detecting an ITD mutation of FLT3 in the aligned sequence information (reads); and
(d) determining to prescribe a targeted anticancer agent when a mutation in the ITD region of FLT3 is detected.

6. A method of diagnosing minimal residual disease (MRD) in an acute myeloid leukemia (AML) patient having an FLT3-ITD mutation, the method comprising:
(a) extracting nucleic acids from a biological sample to obtain sequence information using the primer set according to claim 1 or 2;
(b) aligning the sequence information (reads) to a reference genome database;
(c) detecting an ITD mutation of FLT3 in the aligned sequence information (reads); and
(d) determining that the patient has minimal residual disease (MRD) when the ITD mutation of FLT3 is detected.

7. A method of predicting prognosis of an acute myeloid leukemia (AML) patient having an FLT3-ITD mutation, the method comprising:
(a) extracting nucleic acids from a biological sample to obtain sequence information using the primer set according to claim 1 or 2;
(b) aligning the sequence information (reads) to a reference genome database;
(c) detecting an ITD mutation of FLT3 from the aligned sequence information (reads); and
(d) predicting a prognosis based on a variant allele frequency (VAF) and a length of the detected ITD mutation of FLT3.

8. A method of determining presence of resistance to a tyrosine kinase inhibitor, a therapeutic agent for acute myeloid leukemia (AML), in a patient having an FLT3-ITD mutation, the method comprising:
(a) extracting nucleic acid from a biological sample to obtain sequence information using the primer set according to claim 1 or 2;
(b) aligning the sequence information (reads) to a reference genome database;
(c) detecting a TKD region mutation of FLT3 from the aligned sequence information (reads); and
(d) detecting a TKD region mutation of FLT3 and determining a tyrosine inhibitor to which resistance is present depending on a type of the TKD region mutation.

9. The method according to claim 8, wherein the tyrosine kinase inhibitor is an FLT3 inhibitor.

10. The method according to claim 9, wherein the FLT3 inhibitor comprises at least one selected from the group consisting of sunitinib, lestaurtinib, sorafenib, quizartinib, midostaurin, pacritinib, gilteritinib, crenolanib, and tandutinib.
